# EUROPEAN PATENT APPLICATION

(11) **EP 4 520 283 A2**
(43) Date of publication of application: **12.03.2025**
(21) Application number: 24198802.1
(22) Date of filing: 06.09.2024
(51) Int. Cl.: A61B 18/14, A61B 5/00, A61B 18/00, A61B 34/20

(54) **MEDICAL DEVICE WITH A BASKET AND A HOUSING**

(30) Priority: 08.09.2023 US 202363581435 P; 06.08.2024 US 202418795903
(71) Applicant: Biosense Webster (Israel) Ltd., 2066717 Yokneam (IL)
(72) Inventor: BERMAN, Dror, Irvine, 92618 (US); BAR-TAL, Meir, 2066717 Yokneam (IL); DATTA, Keshava, Irvine, 92618 (US); ZAVALA, Arlene, Irvine, 92618 (US); NGUYEN, Thanh, Irvine, 92618 (US); PHAM, Cuong, Irvine, 92618 (US)
(74) Representative: Carpmaels & Ransford LLP

(57) **Abstract**

The disclosed technology includes an actuator subsystem for a medical probe, including an actuator, a detent, and at least one catch. The actuator is designed to move an end effector between expanded and collapsed configuration. As the actuator is moved, the catches, in conjunction with the detent, provide tactile feedback regarding a position of an end effector of the medical device.

## Description

### CROSS-REFERENCE TO RELATED APPLICATIONS

The application claims benefit of priority to prior filed U.S. Provisional Patent Application No. 63/581,435 filed September 8, 2023 (Attorney Docket No.: BIO6817USPSP1 - 253757.000426), which is hereby incorporated by reference in full herein.

### FIELD

The present technology relates generally to medical devices, and in particular medical probes with electrodes, and further relates to, but not exclusively, medical probes suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference.

Regions of cardiac tissue can be mapped by a catheter to identify the abnormal electrical signals. The same or different medical device can be used to perform ablation. Some example probes include a number of spines with electrodes positioned thereon. These probes must be capable of ablating the inside bell mouth of the pulmonary vein (PV) as well as the entrant surfaces of the PV without excessive pulling back of the spines. Further, these catheters must be able to fit within a 13.5 French (Fr) sheath.

### SUMMARY

There is provided, in accordance with the disclosed technology, an end effector for a medical device. The end effector can include a unitary spine frame. The unitary spine frame can include a proximal end, a distal end comprising a crown, and a plurality of spines integral with the crown. The plurality of spines are configured to bow radially outward from a longitudinal axis and to move between an expanded configuration and a collapsed configuration. The unitary frame is teardrop shaped profile in the collapsed configuration. The teardrop shaped profile has a bulbous distal region that tapers to a proximal region. The bulbous distal region includes the plurality of spines expanding outward with respect to the longitudinal axis from the crown in the proximal direction and the plurality of spines taper toward the longitudinal axis for the smaller proximal region with a transition region between the bulbous region and the trailing region. At least one electrode is disposed on each spine in the bulbous distal region. At least two electrodes are disposed on each spine in the trailing region.

There is further provided, in accordance with the disclosed technology, a housing assembly for a medical device. The housing assembly can include a shell, an actuator, a detent, and a first catch. The shell defines a cavity. The actuator is slidable within the cavity along a longitudinal axis and configured to move an end effector between an expanded configuration and a collapsed configuration. The detent is engaged with one of the shell and the actuator. The first catch is integrated with the other of the shell and the actuator and is configured to selectively engage the detent. The engagement of the detent and the first catch provides tactile feedback regarding a collapsed configuration, an intermediate configuration, or an expanded configuration of the end effector.

There is further provided, in accordance with the disclosed technology, a medical device. The medical device can include a first shaft, an end effector, and a housing assembly. The first shaft extends along a longitudinal axis and is attached to the actuator. The end effector is attached to the first shaft at a proximal end of the end effector and is configured to move between an expanded configuration, an intermediate configuration, and a collapsed configuration. The housing assembly can include an actuator, a detent, and a first catch. The actuator is attached to the first shaft and is slidable along the longitudinal axis. Sliding movement of the actuator slides the first shaft and the proximal end of the end effector. The first catch is configured to selectively engage with the detent. The first catch is configured to provide tactile feedback that the end effector is in one of the expanded configuration, the intermediate configuration, and the collapsed configuration.

### BRIEF DESCRIPTION OF THE DRAWINGS

FIG. 1 is a schematic pictorial illustration of a medical system including a medical device with a distal end that includes a medical probe with electrodes, in accordance with the disclosed technology;
FIG. 2 is a schematic pictorial illustration showing a perspective view of a medical device, with portions of an elongated shaft cut away, in accordance with the disclosed technology;
FIG. 3 is a schematic pictorial illustration showing a detailed schematic view of a portion of a handle, in accordance with the disclosed technology;
FIG. 4 is a schematic pictorial illustration showing a detailed cross-section view of the handle and a portion of an actuator subsystem, in accordance with the disclosed technology;
FIG. 5 is a schematic pictorial illustration showing a perspective view of an end effector of the medical device, in accordance with the disclosed technology;
FIG. 6 is a schematic pictorial illustration showing the medical device from a distal end thereof, in accordance with the disclosed technology;
FIG. 7 is a schematic pictorial illustration of a side elevation view of the end effector disposed within a sheath, in accordance with the disclosed technology;
FIG. 8 is a schematic pictorial illustration of a side elevation view of a unitary spine frame, in accordance with the disclosed technology;
FIG. 9 is a schematic pictorial illustration of a detail side cross-sectional view of the distal end of the end effector, in accordance with the disclosed technology;
FIG. 10 is a schematic pictorial illustration of a perspective view of the distal end of the end effector, in accordance with the disclosed technology;
FIG. 11 is a schematic pictorial illustration of an annulus of the end effector, in accordance with the disclosed technology;
FIG. 12 is a schematic pictorial illustration of a detail perspective view of the proximal end of the of the end effector with a spine retention collar removed, in accordance with the disclosed technology;
FIG. 13 is schematic pictorial illustration showing the end effector in a position just after actuation of the actuator subsystem is initiated, in accordance with the disclosed technology;
FIG. 14 schematic pictorial illustration showing the end effector in a position intermediate a collapsed configuration and an expanded configuration, in accordance with the disclosed technology; and
FIG. 15 is a schematic pictorial illustration showing the end effector in the expanded configuration, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

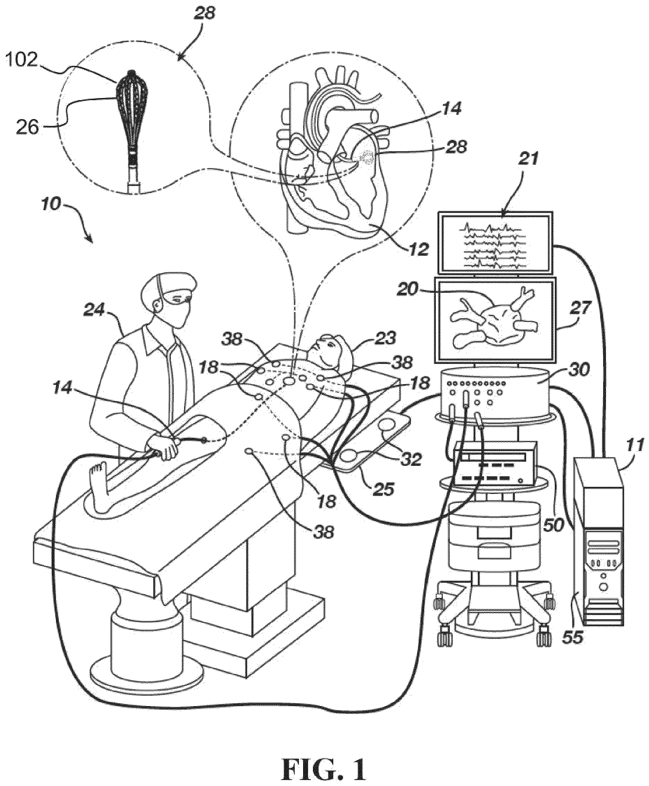

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example medical probe, e.g., a catheter 14, that is configured for sensing IEGM is illustrated herein. Physician 24 brings a distal tip of catheter 14 (i.e., a basket assembly 28 in this case) into contact with the heart wall for sensing a target site in heart 12. A catheter 14 with a distal basket assembly can be referred to as a basket catheter. For ablation, physician 24 would similarly bring a distal end of an ablation catheter to a target site for ablating.

Catheter 14 is an exemplary catheter that includes one and preferably multiple electrodes 26 optionally distributed over a plurality of spines 104 at basket assembly 28 and configured to sense the IEGM signals. Catheter 14 may additionally include a position sensor embedded in or near basket assembly 28 for tracking position and orientation of basket assembly 28. Optionally and preferably, position sensor is a magnetic based position sensor including three magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of basket assembly 28 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTOTM 3 System, available from Biosense Webster, Inc., 31 Technology Drive, Suite 200, Irvine, CA 92618 USA.

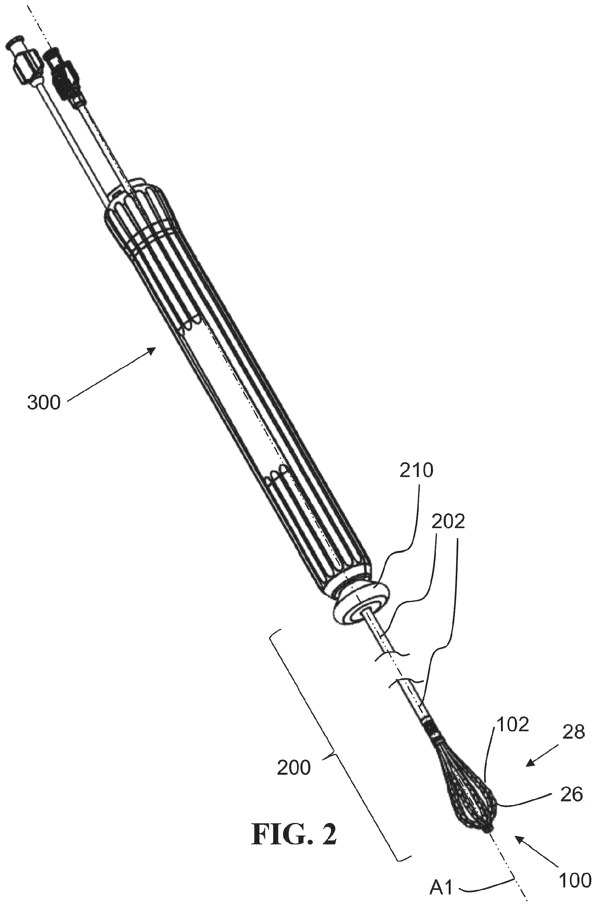

FIG. 2 is a schematic pictorial illustration showing a perspective view of a medical device 14 with electrodes 26. The medical device 14 includes, at its distal tip 28, an end effector 100. The end effector 100 in the presently described example takes the form of a basket assembly that includes one or more spines 104. In some examples, the one or more spines 104 may be formed integrally with a crown 108 so as to form a unitary/monolithic spine frame 102.

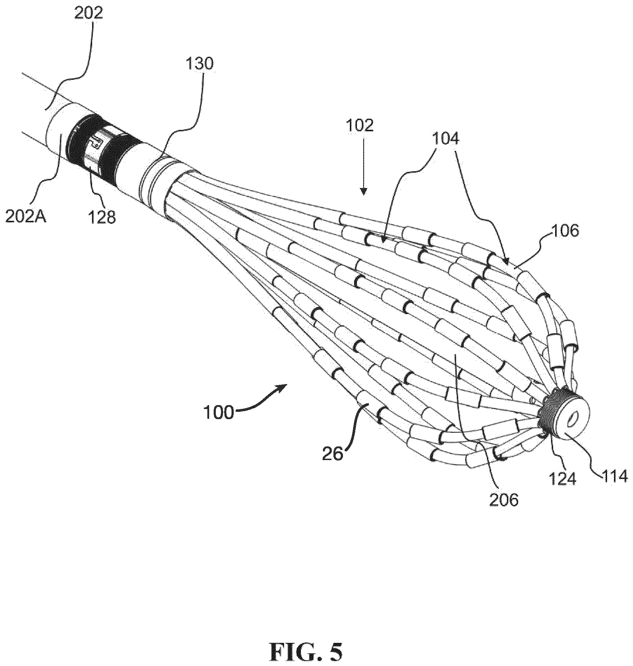

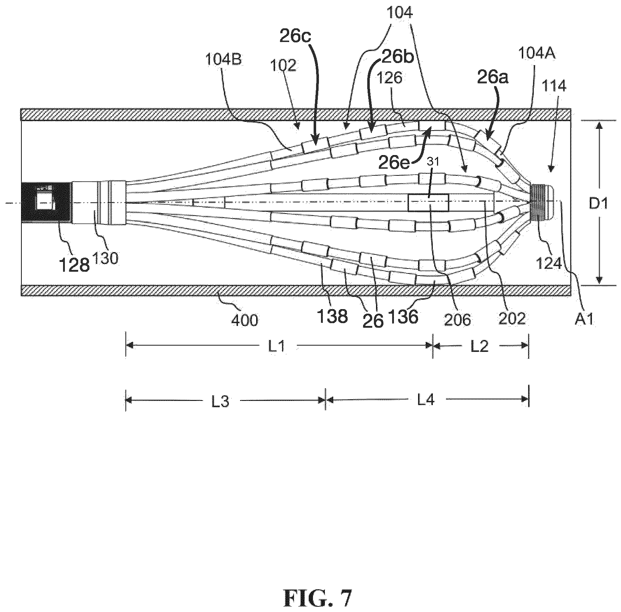

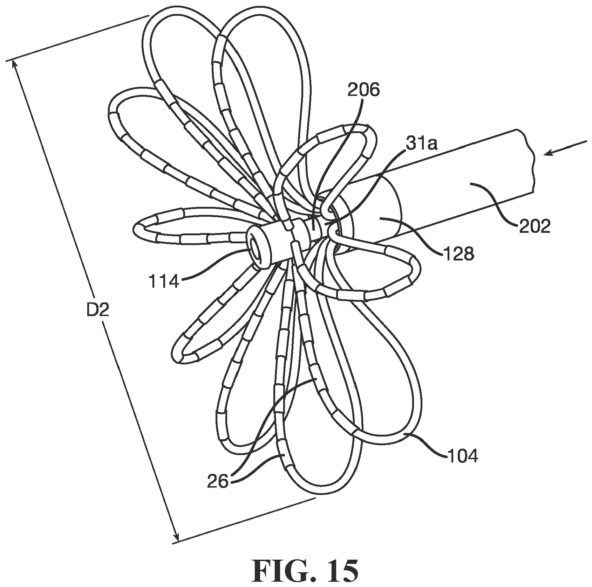

The one or more spines 104 are movable between an expanded configuration and a collapsed configuration. Such movement can be achieved by an actuator subsystem 200 which includes, among other elements, an actuator 210 and an elongated shaft 200, and is described further below. The elongated shaft 202 connects the end effector 100 to a handle that, in use, the operator 24 can manipulate. Elongated shaft 202 can also sometimes be referred to as actuator shaft when shaft 202 is fixed to the distal end of the basket and connected to an actuator at the proximal handle 300 for translation along the longitudinal axis A1. FIG. 7 is a schematic pictorial illustration showing a side view of the end effector 100 within a sheath 400 in the collapsed configuration. In the expanded configuration (FIG. 15) as well as the collapsed configuration, one or more spines 104 bow radially outwardly. As seen in FIGs. 2 and 5, the spine frame 102 can be teardrop shaped profile in form in the collapsed configuration. The spines 104, elongated shaft 202, and handle 300 are arranged generally along a longitudinal axis A1 when the elongated shaft 210 is unbent.

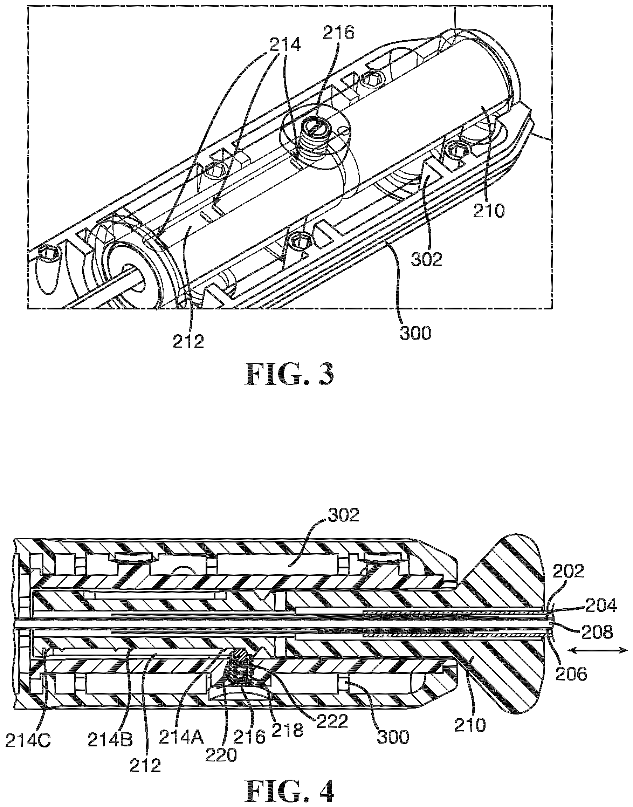

FIG. 3 is a schematic pictorial illustration showing a detailed schematic view of a portion of the handle 300. The handle 300 is also referred to herein as a housing assembly. As shown, the handle 300 may have a clamshell design that defines a cavity 302. In FIG. 3, one portion of the clamshell is removed to schematically depict an exemplary interior thereof. As shown, a portion of the actuator subsystem 200 is housed within the handle 300 and extends in parallel therewith along the longitudinal axis A1.

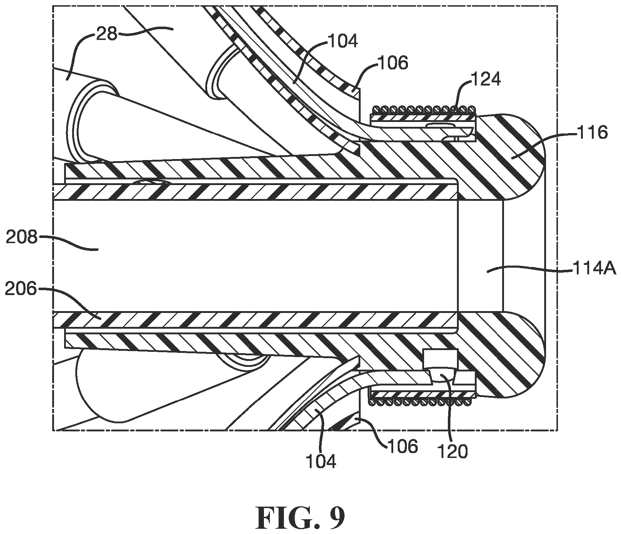

FIG. 4 is a schematic pictorial illustration showing a detailed cross-section view of the handle 300 and a portion of the actuator subsystem 200. Further to the above, an actuator 210 can take the form of a push/pull rod with an enlarged head (right side in FIG. 4) for an operator 24 to manipulate. A proximal end of the elongated shaft 202, which is also referred to herein as a first elongated shaft, is connected to a distal end of the actuator 210 in a manner that allows in unison translation of both when the actuator 210 is manipulated, which is discussed in greater detail below. A distal end of the elongated shaft 202 (FIG. 5) can be connected to the end effector 100 via a collar 102A. The elongated shaft 202 defines a lumen 204 therethrough which receives another elongated shaft 206, which is also referred to herein as a second elongated shaft 206. The second elongated shaft 206 can be routed through the handle 300 and first elongated shaft 202 and affixed, at its distal end, to a distal end of the end effector 100 (FIG. 9). The first elongated shaft 202 is slidable relative to the second elongated shaft 206, which is discussed in greater detail below. The second elongated shaft 206 can be used, in part, to route irrigation to the end effector 100 though its lumen 208. For example, the second elongated shaft 206 can be provided with irrigation holes at a distal portion thereof. Both the first elongated shaft 202 and second elongated shaft 206 can be formed from a flexible, biocompatible electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response.

With continued reference to FIGs. 3 and 4, a pill-shaped relief 212 can be formed in a wall of the actuator 210 that is disposed within the handle 300. Within the relief 212 are a plurality of protrusions 214 for providing tactile feedback to the operator 14, which is discussed in greater detail below. For example, the plurality of protrusions/ribs 214 can include a first set of protrusions/ribs 214A, which function as a first catch, a second set of protrusions/ribs 214B, which function as a second catch, and a third set of protrusions/ribs 214C, which function as a third catch. Of course, any number of catches may be employed without departing from the spirit and scope of the present disclosure. The protrusions 214 are used in conjunction with a detent 216 that can be connected to or otherwise integrated with the housing 300. In the presently depicted example, the detent 216 includes a hollow pin 216, with a spring 222 provided within a cavity of the pin 216 to bias a ball 220 away from that cavity and towards the relief 212 and protrusions 214. The detent 216 can be movable towards and away from the protrusions/catches 214 in a direction perpendicular to the longitudinal axis A1, which has the effect of adjusting a force applied by the detent 216 to the protrusions 214. This can be achieved, for example, by threading the detent 216 with the housing 300.

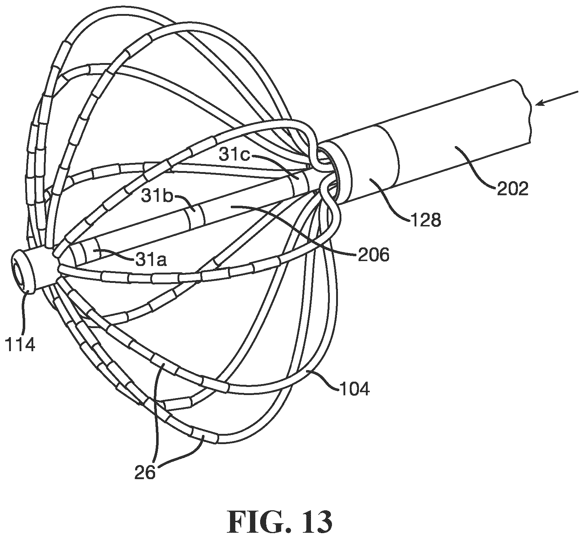

Of course, other configurations of this portion of the actuator subsystem may be employed. For example, a different structural configuration of the detent 216 may be employed. Further, as an alternative, the protrusions 214 may be provided on a portion of the handle 300 and the detent 216 be connected to the actuator 210. Operation of the actuator subsystem is described in greater detail below with respect to FIGs. 13-15.

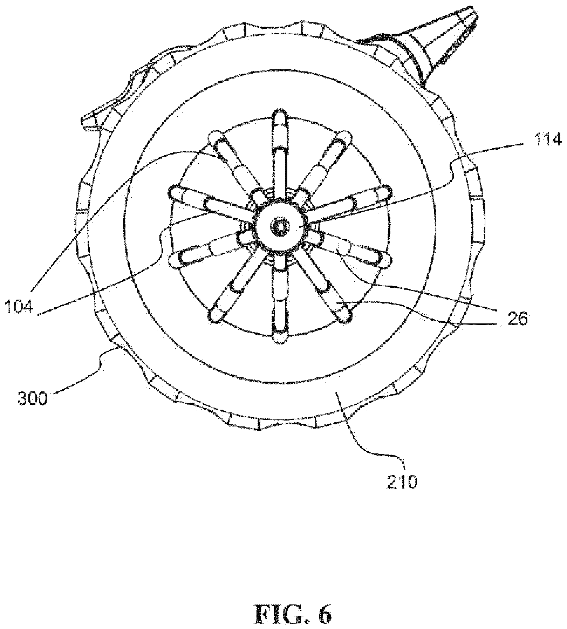

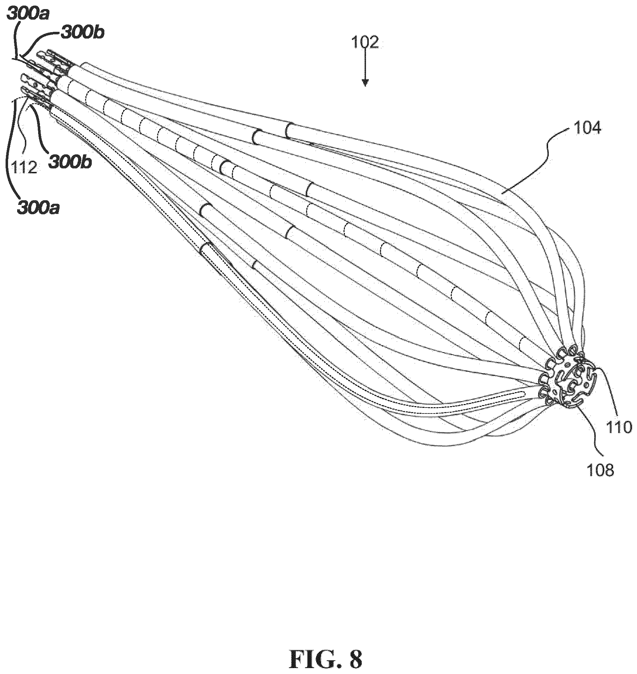

FIG. 5 is a schematic pictorial illustration showing a perspective view of an end effector 100 of the medical device in accordance with the disclosed technology. FIG. 6 is a schematic pictorial illustration showing the medical device from a distal end thereof. FIG. 7 is a schematic pictorial illustration of a side elevation view of the end effector 100 disposed within a sheath 400. FIG. 8 is a schematic pictorial illustration of a side elevation view of the unitary spine frame 102. With reference to FIGs. 5-8, and as discussed above, a unitary spine frame 102 may be employed. The frame 102 may include one or more flexible spines 104 that are connected to the first elongated shaft 202 at a proximal section 104B thereof and the second elongated shaft 206 at a distal section 104A thereof. During a medical procedure, the operator 24 can deploy the end effector 100 from a sheath 400 having a diameter D1 (for example, 12 Fr or 13.5 Fr in diameter, see FIG. 7). by extending the shafts 202, 206 through the sheath 400, causing the end effector 100 to exit the sheath 400, at which point it can be transitioned to the expanded configuration (discussed in greater detail below). Spines 104 may have elliptical (e.g., circular) or rectangular (that may appear to be flat) cross-sections, and include a flexible, resilient material (e.g., a shape-memory alloy such as nickel-titanium, also known as Nitinol) forming a strut.

In FIG. 7, it can be seen that the plurality of spines define a tear drop shaped profile with the spines expanding outward from the longitudinal axis in the proximal direction. It can be seen that the spines (126 and 136) expand outward for a length L2 at which point the expansion of the spines stops and the spines become essentially tangent to the inside surface of tubular member 400. This profile of the spines over L2 is considered to be a "bulbous region" of the end effector. The spines then taper inwardly (towards the longitudinal axis) over a length of L1 extending towards the proximal near 130. This profile over L1 is considered a "trailing region". Between the "bulbous region" and the "trailing region" is a "transition region" in which the spine becomes tangent to the inside surface of tubular member 400. We have devised a unique arrangement of electrodes for bulbous and trailing regions for what is believed to be the optimum ablation electrode arrangement in pulmonary vein ablation procedures. In particular, at least one electrode must be placed in at least one spine in the bulbous region with at least two electrodes must be placed in the trailing region. As well, every other spine in the plurality of spines must have one electrode disposed in the transition region (i.e., the region of maximum outer diameter defined by the spine being contiguous to the tubular inner surface of member 400). In the exemplary spine 126, it can be seen that this requirement of at least one electrode 26 in the bulbous region is satisfied by electrode 26a and the requirement of at least two electrodes disposed in the trailing region is satisfied by electrodes 26b and 26c. The requirement of one electrode over the transition region is satisfied by electrode 26e.

Referring back to FIG. 8, it can be seen the location of each of the spine 104 and therefore the overall shape of the basket can be determined via the use of a magnetic field sensor in the form of a multiple loops formed a helically looping of a wire having two leads 300a and 300b in which the wire loop (shown as dashed line) around the spine in a helix wrapping around each of the plurality of spines. Alternatively, a flat loop can also be defined by a single wire with two leads 300a and 300b. Each spine would have the wire loop in the form of a helix (middle spine) or the flat loop shown in the lower spine. Support for the magnetic location sensor and the shape determination of this sensor can be understood from US Patent Publication Nos. US20180344202A1, US20220265194A1 or US Patent No. US10687761B2 which are each incorporated by reference with a copy provided in the Appendix of priority application no. 63/581,435.

As will be appreciated, the spines 104 can be electrically isolated from the electrode 26 to prevent arcing from the electrodes 26 to the spines 104. For example, insulative jackets can be positioned between the spine(s) 104 and the electrode(s) 26, but one of skill in the art will appreciate that other insulative coverings are contemplated. For example, an insulative coating can be applied to the spines 104, the electrodes 26, or both. The insulative jackets can be made from a biocompatible, electrically insulative material such as polyamide-polyether (Pebax) copolymers, polyethylene terephthalate (PET), urethanes, polyimide, parylene, silicone, etc. In some examples, insulative material can include biocompatible polymers including, without limitation, polyetheretherketone (PEEK), polyglycolic acid (PGA), poly(lactic-co-glycolic acid) copolymer (PLGA), polycaprolactive (PCL), poly(3-hydroxybutyrate-co-3-hydroxyvalerate) (PHBV), poly-L-lactide, polydioxanone, polycarbonates, and polyanhydrides with the ratio of certain polymers being selected to control the degree of inflammatory response. Insulative jackets 106 may also include one or more additives or fillers, such as, for example, polytetrafluoroethylene (PTFE), boron nitride, silicon nitride, silicon carbide, aluminum oxide, aluminum nitride, zinc oxide, and the like.

In embodiments described herein, electrodes 26 can be configured to deliver ablation energy (IRE and/or RF) to tissue in heart 12. In addition to using electrodes 26 to deliver ablation energy, the electrodes 26 can also be used to determine the location of the end effector 100 and/or to measure a physiological property such as local surface electrical potentials at respective locations on tissue in heart 12. The electrodes 26 can be biased such that a greater portion of the electrode 26 faces outwardly from the end effector 100 such that the electrodes 26 deliver a greater amount of electrical energy outwardly away from the end effector 100 (i.e., toward the heart 12 tissue) than inwardly toward the end effector 100.

Examples of materials ideally suited for forming electrodes 26 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 12.

As shown particularly in FIGs. 5 and 6, at least three electrodes 26 can be attached to each spine 104. For example, four electrodes 26 can be attached to each spine 104, with at least ten spines 104 radially disposed about the end effector 100 such that 30-40 electrodes, that are sized and designed in conjunction with the second elongated shaft 206, can be provided on the basket assembly while still fitting within a 12 Fr or a 13.5 Fr sheath. As shown particularly in FIGs. 5-7, the electrodes 26 are staggered relative to electrodes 26 on adjacent spines 104 along the longitudinal axis A1. In other examples, the electrodes 26 do not necessarily need to be staggered to still fit within a 12 Fr or a 13.5 Fr sheath. Also, as seen particularly in FIG. 7, the electrodes 28 are also generally weighted/positioned on the spines 104 towards the distal section 104A such that there are no electrodes 28 in the proximal section 104B of the end effector 100. A reference electrode 31 can be disposed at a region between L1 and L2 in Fig. 7 so that reference electrode is generally equi-center to the electrodes 28 disposed between regions defined by L1 and L2.

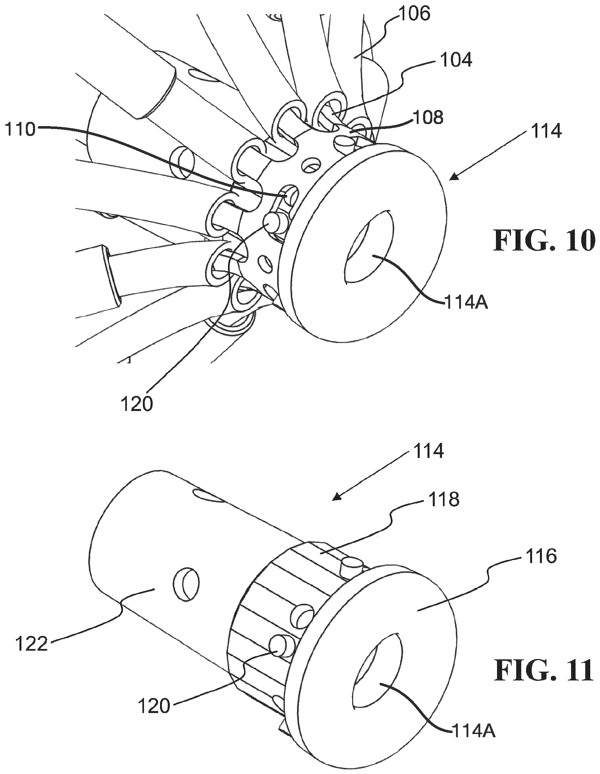

FIG. 9 is a schematic pictorial illustration of a detail side cross-sectional view of the distal end of the end effector 100. FIG. 10 is a schematic pictorial illustration of a perspective view of the distal end of the end effector 100, with the coil 124 removed for clarity. FIG. 11 is schematic pictorial illustration of an annulus 114 of the end effector 100. Making reference to FIGs. 9-11, and as discussed above, the second elongated shaft 206 is connected to the distal end of the end effector. Specifically, the end effector 100 includes an annulus 114 having a lumen 114A within which the second elongated shaft 206 is affixed. The annulus includes a rounded atraumatic tip 116, a friction ring midsection 118, locking projections 120 disposed radially around the friction ring midsection 118, and an annulus shaft 122 opposite the atraumatic tip 116. The annulus lumen 114A is axially aligned with the second elongated shaft lumen 208 such that various medical devices can be extended therethrough when in use, such as a mapping catheter or a guidewire. The annulus 114 is connected to the distal end 108 of the unitary spine frame 102 and extends therethrough, the distal end 108 also referred to herein as the crown 108. As seen particularly in FIG. 10, the crown 108 is provided with a plurality of cutouts 110 radially disposed about its circumference, and within which the locking projections 120 engage to lock the crown 108 to the annulus 114. Additionally, the friction ring midsection 118 frictionally engages an inner annular surface of the crown 108 to aid in preventing dislodgement. The annulus shaft 122 engages an outer surface of the second elongated shaft 206.

With reference being made to FIGs. 7 and 9, integrated with or connected to the annulus 114 is a first coil 124. The first coil 124 can be configured to generate a current when subjected to a magnetic field. In some examples, the first coil 124 can comprise a single axis sensor (SAS). In other examples, the coil 124 can comprise a dual axis sensor (DAS) or a triple axis sensor (TAS). The first coil 124 can comprise a conductive material wound in a coil or a coil formed into a flexible circuit. The first coil 124 can comprise electrical leads for conduction of current induced on the first coil 124 to the patient interface unit 30. As will be appreciated, by attaching a first coil 124 to the distal end of the end effector 100, it is possible to detect a position thereof. In this way, a physician 24 can more accurately determine the position of the distal end of the end effector 100 before applying ablative energy to tissue.

Also provided on the end effector 100 are one or more second coils 126 (FIG. 7) disposed within the jacket(s) 106 and/or the spine(s) 104. In some examples, the second coil(s) 126 can comprise a SAS. In other examples, the second coil(s) 126 can comprise a DAS or a TAS. The second coil(s) 126 can comprise a conductive material wound in a coil or a coil formed into a flexible circuit. The second coil(s) 126 can comprise electrical leads for conduction of current induced on the second coil(s) 126 to the patient interface unit 30. As will be appreciated, by attaching a second coil(s) 126 to one or more of the spines 104, it is possible to detect a position thereof. In this way, like with the first coil 124, a physician 24 can more accurately determine the position of the spine(s) 104 of the end effector 100 before applying ablative energy to tissue.

Further provided on or adjacent the end effector 100 at its proximal end is a third coil 128 (FIGs. 5 and 7) sandwiched between a collar 202A of the first elongated shaft 202 and a spine retention collar 130. In some examples, the third coil 128 can comprise DAS or a TAS. In other examples, the third coil 128 can comprise a SAS. The third coil 128 can comprise a conductive material wound in a coil or a coil formed into a flexible circuit. The third coil 128 can comprise electrical leads for conduction of current induced on the third coil 128 to the patient interface unit 30. As will be appreciated, by attaching a third coil 128 at a proximal end of the end effector 100, it is possible to detect a position thereof. In this way, like with the first coil 124 and the second coil(s) 126, a physician 24 can more accurately determine the position of the proximal end of the end effector 100 before applying ablative energy to tissue.

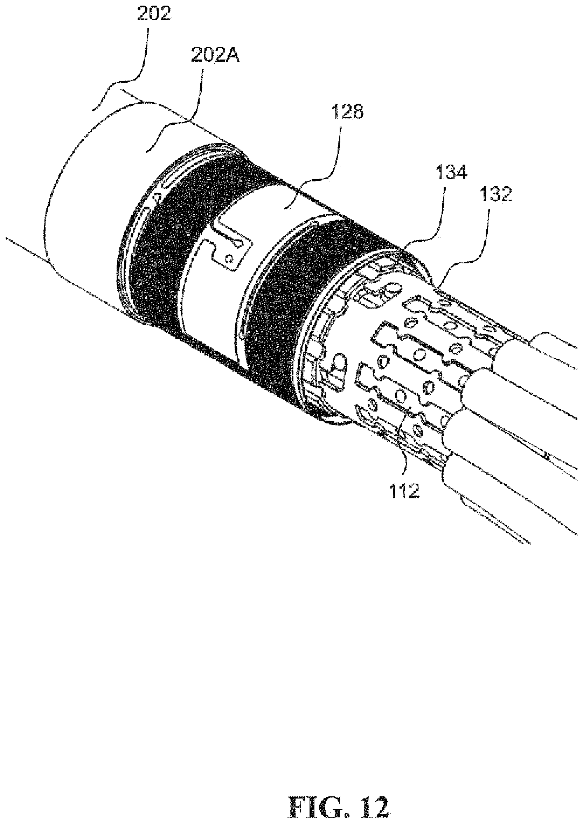

FIG. 12 is a schematic pictorial illustration of a detail perspective view of the proximal end of the of the end effector 100 with the spine retention collar 130 removed, for clarity. Proximal ends 112 of the spines 104 have tabs that are shaped to engage with a spine coupler 132. The spine coupler connects to an irrigation manifold 134 that provides irrigation to the end effector 100. In the illustrated example, the engagement can be employed via a similar manner that the annulus 114 and crown 108 interlock.

With particular reference to FIGs. 4, 7, and 13-15, the actuator subsystem 200 will now be described in greater detail. With the teardrop shaped profile geometry of the spine frame 102 in the collapsed configuration, the spine frame 102 has a largest diameter D1 at a first location 136 along the spines 104 that is offset from a center of the spine frame 102. More specifically, the first location 136 is towards the distal section 104A of the end effector 100 with a distance of L1 from a proximal end of the spines 104 and/or the crown 108 and a distance of L2 to the distal end of the spines 104. To allow for maneuverability within the heart, the sum of L1 and L2 is minimized in the disclosed technology. For example, the sum of L1 and L2 can fall within the range of 30-50 millimeters. In the collapsed configuration, the detent 216 engages with the first catch 214A. In some examples, the detent 216 can be adjusted to lock it with the first catch 214A (as well as the other catches 214B, 214C). This feature allows the operator 24 to maintain a precise intended geometry of the end effector 100, the geometry changing as the end effector 100 is expanded.

FIG. 13 is schematic pictorial illustration showing the end effector 100 in a position just after actuation of the actuator subsystem 200 is initiated. This cone shape allows for easy placement within pulmonary veins of the patient 23. More specifically, as the actuator 210 is pulled along the longitudinal axis A1 with sufficient force towards the end effector 100 to protrude further from the handle 300, the detent 216 leaves engagement with the first catch 214A, providing tactile feedback to the operator 24 that the end effector 100 is no longer in the collapsed configuration. As this movement of the actuator 210 occurs, the spines 104 begin to bow further radially outwards (relative to the collapsed configuration). This action is achieved by sliding movement of the first elongated shaft 202 (slid due to its connection to the actuator 210) relative to the second elongated shaft 206 in a telescopic manner such that the first elongated shaft 202 rides along the second elongated shaft 206.

Instead of the reference electrode 31 in FIG. 7, three reference electrodes 31a, 31b and 31c can be disposed on actuator shaft 206. By having three reference electrodes 31, this example ensures that at least one reference electrode 31 is always exposed to the blood medium so that far-field signals (e.g., noise) can be sensed or collected for noise cancellation with respect to near field signals collected by the electrodes 28 in tissue contact.

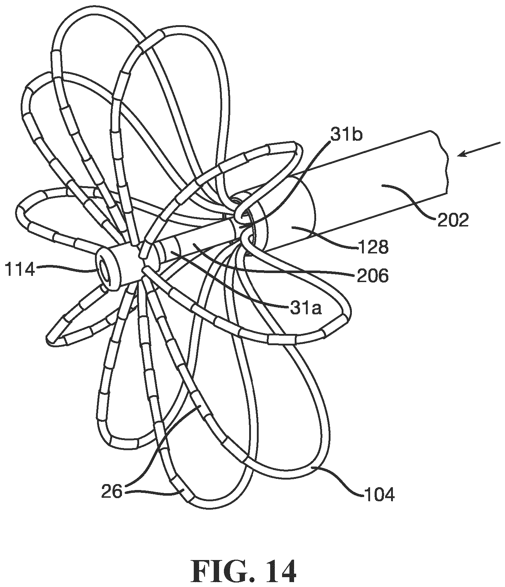

FIG. 14 schematic pictorial illustration showing the end effector 100 in a position intermediate the collapsed configuration and the expanded configuration. In this intermediate configuration, the spines 104 bow further radially outwardly as the first elongated shaft slides closer to the annulus 114. Additionally, the detent 216 engages the second catch 214B, providing tactile feedback to the operator 24 that the end effector is in the intermediate configuration. As touched on above, the detent 216 can be provided as threadably adjustable such that the detent 216 can be moved towards the catch 214B to be locked in the intermediate position, should the operator 24 need to maintain this position for a period of time or the expanded configuration is not needed at a particular treatment area. To unlock and/or continue movement of the actuator 210, the detent 216 can be adjusted away from the catch 214B to reduce the force that it applies to the second catch 214B, and sufficient force from the operator 24 enables the detent 216 to leave engagement with the second catch 214B.

FIG. 15 is a schematic pictorial illustration showing the end effector 100 in the expanded configuration, with the spines 104 being fully bowed outwards/expanded, with a largest diameter D2 that is greater than the largest diameter D1 in the collapsed configuration. In this position, a distal end of the first elongated shaft 202 is disposed proximal the annulus 114 and the spines 104 resembles flower petals. In some examples, the proximal end and distal end of the end effector 100 can abut or substantially abut one another in the expanded configuration (i.e., there is no space separating the ends or only a few (e.g., 1-3) millimeter gap separating them). To form the teardrop shape, the proximal and distal ends of the end effector can be spaced approximately 25mm, for example. Once the actuator 210 has reached the extent of its travel, the detent engages the third catch 214C, thus providing tactile feedback to the operator 24 that the end effector 100 is fully expanded. Like described above with respect to the other catches, the operator 24 can also manipulate the detent 216 to lock with the third catch 214C.

In the expanded configuration, an approximate point along the spines 104 of the widest diameter D2 is the second location 138 depicted in FIG. 7. With the petal shaped geometry of the spine frame 102 in the expanded configuration, the largest diameter D2 at the second location 138 along the spines 104 is closer to the proximal end of the end effector 100 than the first location 136. More specifically, the second location 136 has a distance of L3 (less than L1) from a proximal end of the spines 104 and a distance of L4 (greater than L2) to the distal end of the spines 104. In some examples, a ratio of L1 to L2 is approximately 10 to 3 and a ratio of L3 to L4 is approximately 1 to 1. In some examples, a ratio of the sum of L1 and L2 (and, thus, L3+L4 as well) to D2 can be approximately 1.2:1. In this position, due to the positioning of the electrodes 26 towards the distal end of the spines 104, the electrodes 26 substantially face forwards (i.e., towards the distal end of the end effector) and are able to ablate entrant surfaces of the PV without excessive pulling back at the distal tip of the end effector 100.

As will be appreciated, operation of the actuator 210 in the direction opposite that of the one described collapses the end effector 100 back into the collapsed configuration, with the catches 214A, 214B, 214C providing tactile feedback along the actuator's travel path to provide an indication of the shape configuration of the end effector 100. As mentioned above, while only three catches 204 are described, any number can be employed without departing from the spirit and scope of the present disclosure.

Additionally, alternative examples of the actuator 210 include a pull wire that can be employed in a similar manner as described above. Rather than pushing the elongated shaft 202, the pull wire can be routed around, for example, the annulus 214 and connected to the proximal end of the end effector 100, thus enabling similar shape configurations described above.

The disclosed technology described herein can be further understood according to the following clauses:
Clause 1. An end effector for a medical device, the end effector comprising: a unitary spine frame comprising: a proximal end, a distal end comprising a crown, and a plurality of spines integral with the crown, the plurality of spines being configured to bow radially outward from a longitudinal axis and to move between an expanded configuration and a collapsed configuration, the unitary frame defining a teardrop shaped profile in the collapsed configuration, the teardrop shaped profile having a bulbous distal region that tapers to a proximal region, the bulbous distal region including the plurality of spines expanding outward with respect to the longitudinal axis from the crown in the proximal direction and the plurality of spines tapering toward the longitudinal axis for the smaller proximal region with a transition region between the bulbous region and the trailing region; at least one electrode being disposed on each spine in the bulbous distal region; and at least two electrodes being disposed on each spine in the trailing region.
Clause 2. The end effector of clause 1, each spine being movable such that: in the collapsed configuration, a widest diameter of the plurality of spines is disposed at a first location along the longitudinal axis that is more proximal to the crown than the proximal end, and in the expanded configuration, a widest diameter of the plurality of spines is disposed at a second location along the longitudinal axis that is spaced from the first location.
Clause 3. The end effector of clause 2, the second location being spaced from the first location in a direction towards the proximal end.
Clause 4. The end effector of any one of clauses 2-3, the proximal end and the first location being spaced by a first length, and the first location and the crown being spaced by a second length, a ratio of the first length to the second length being approximately 10 to 3.
Clause 5. The end effector of any one of clauses 2-4, the proximal end and the second location being spaced by a third length, and the second location and the crown being spaced by a fourth length, a ratio of the third length to the fourth length being approximately 1 to 1.
Clause 6. The end effector of any one of clauses 1-5, the plurality of spines being at least ten spines.
Clause 7. The end effector of any one of clauses 1-6, further comprising at least three electrodes attached to each spine.
Clause 8. The end effector of clause 7, the at least three electrodes of each spine being attached towards the distal end.
Clause 9. The end effector of any one of clauses 7-8, the at least three electrodes of one spine being staggered relative to the at least three electrodes of an adjacent spine.
Clause 10. The end effector of any one of clauses 1-9, further comprising an annulus running at least partially through the crown and covering a terminal end of the crown.
Clause 11. The end effector of clause 10, the annulus having a rounded outermost surface.
Clause 12. The end effector of any one of clauses 10-11, the annulus comprising at least one annulus projection that engages in at least one slot in the crown.
Clause 13. The end effector of any one of clauses 1-12, further comprising a first single axis sensor attached to the crown.
Clause 14. The end effector of any one of clauses 1-13, further comprising a second single axis sensor disposed within one spine of the plurality of spines.
Clause 15. The end effector of any one of clauses 1-14, further comprising a dual axis sensor, a flex circuit, or a triple axis sensor disposed proximal the proximal end.
Clause 16. The end effector of any one of clauses 1-16, a proximal end of each spine comprising a tab that engages in a complementarily shaped recess in a spine coupler.
Clause 17. The end effector of any one of clauses 1-16, configured to fit within a 13.5 French sheath.
Clause 18. The end effector of any one of the clauses 1-17, further comprising an actuator shaft connected to a distal portion of the spines and coupled to an actuator and at least one reference electrode disposed on the actuator shaft.
Clause 19. The end effector of clause 18, wherein the at least one reference electrode comprises three reference electrodes disposed at three distinct locations on the actuator shaft.
Clause 20. The end effector of any one of clauses 1-16, further comprising a wire loop extending along each of the plurality of spines to define a magnetic field location sensor for each spine.
Clause 21. The end effector of clauses 1-16, further comprising at least one wire looping helically about each of the plurality of spines to define a magnetic field location sensor.
Clause 22. A housing assembly for a medical device, the housing assembly comprising: a shell defining a cavity; an actuator slidable within the cavity along a longitudinal axis and configured to move an end effector between an expanded configuration and a collapsed configuration; a detent engaged with one of the shell and the actuator; and a first catch integrated with the other of the shell and the actuator and configured to selectively engage the detent, the engagement of the detent and the first catch providing tactile feedback regarding a collapsed configuration, an intermediate configuration, or an expanded configuration of the end effector.
Clause 23. The housing assembly of clause 22, further comprising a second catch integrated with the other of the shell and the actuator and configured to selectively engage the detent.
Clause 24. The housing assembly of clause 23, further comprising a third catch integrated with the other of the shell and the actuator and configured to selectively engage the detent.
Clause 25. The housing assembly of clause 24, the first catch being configured to provide tactile feedback that the end effector is in the collapsed configuration, the second catch being configured to provide tactile feedback that the end effector is in the intermediate configuration, and the third catch being configured to provide tactile feedback that the end effector is in the expanded configuration.
Clause 26. The housing assembly of any one of clauses 22-25, the detent comprising a ball biased towards the first catch.
Clause 27. The housing assembly of any one of clauses 22-26, the detent being movable towards and away from the first catch in a direction perpendicular to the longitudinal axis to adjust a force applied by the detent to the first catch.
Clause 28. The housing assembly of clause 27, the detent being threaded to move the detent towards and away from the first catch.
Clause 29. The housing assembly of any one of clauses 22-25, the actuator partially protruding from the cavity.
Clause 30. The housing assembly of any one of clauses 22-29, the actuator comprising a push/pull rod or a pull wire.
Clause 31. A medical device comprising: a first shaft extending along a longitudinal axis and attached to the actuator; an end effector attached to the first shaft at a proximal end of the end effector and configured to move between an expanded configuration, an intermediate configuration, and a collapsed configuration; and a housing assembly comprising: an actuator attached to the first shaft and slidable along the longitudinal axis, sliding movement of the actuator sliding the first shaft and the proximal end of the end effector; a detent; and a first catch configured to selectively engage with the detent, the first catch being configured to provide tactile feedback that the end effector is in one of the expanded configuration, the intermediate configuration, and the collapsed configuration.
Clause 32. The medical device of clause 31, the end effector comprising a plurality of spines, each spine comprising a plurality of electrodes attached thereto.
Clause 33. The medical device of any one of clauses 31-32, the end effector being teardrop shaped.
Clause 34. The medical device of any one of clauses 31-33, further comprising a second catch configured to selectively engage the detent, the second catch being configured to provide tactile feedback that the end effector is in another of the expanded configuration, the intermediate configuration, and the collapsed configuration.
Clause 35. The medical device of clause 34, further comprising a third catch configured to selectively engage the detent, the second catch being configured to provide tactile feedback that the end effector is in another of the expanded configuration, the intermediate configuration, and the collapsed configuration.
Clause 36. The medical device of any one of clauses 31-35, the first catch comprising a pair of ribs defined on a surface of the actuator.
Clause 37. The medical device of any one of clauses 31-36, the housing assembly comprising a shell, the detent being threaded onto the shell.
Clause 38. The medical device of any one of clauses 31-37, further comprising a second shaft extending along the longitudinal axis and attached to the housing assembly and a distal end of the end effector.
Clause 39. The medical device of clause 38, the second shaft extending through the first shaft.
Clause 40. The medical device of any one of clauses 38-39, the second shaft defining a lumen and comprising one or more irrigation holes.
Clause 41. The medical device of any one of clauses 38-40, the proximal end of the end effector being configured to slide along the second shaft when moving between the expanded configuration, the intermediate configuration, and the collapsed configuration.
Clause 42. The medical device of any one of clauses 38-41, the proximal end of the end effector and the distal end of the end effector substantially abutting one another in the expanded configuration.
Clause 43. The medical device of clause 32, further comprising a wire disposed on at least one spine of the plurality of spines to define at least one loop of a magnetic field sensor.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. An end effector for a medical device, the end effector comprising:
a unitary spine frame comprising: a proximal end, a distal end comprising a crown, and a plurality of spines integral with the crown, the plurality of spines being configured to bow radially outward from a longitudinal axis and to move between an expanded configuration and a collapsed configuration, the unitary spine frame defining a teardrop shaped profile in the collapsed configuration, the teardrop shaped profile having a bulbous distal region that tapers to a trailing proximal region, the bulbous distal region including the plurality of spines expanding outward with respect to the longitudinal axis from the crown in a proximal direction and the plurality of spines tapering toward the longitudinal axis for the smaller proximal region with a transition region between the bulbous distal region and the trailing proximal region;
at least one electrode being disposed on each spine in the bulbous distal region; and
at least two electrodes being disposed on each spine in the trailing proximal region.

2. The end effector of claim 1, each spine being movable such that:
in the collapsed configuration, a widest diameter of the plurality of spines is disposed at a first location along the longitudinal axis that is more proximal to the crown than the proximal end, and
in the expanded configuration, a widest diameter of the plurality of spines is disposed at a second location along the longitudinal axis that is spaced from the first location,
optionally the second location being spaced from the first location in a direction towards the proximal end.

3. The end effector of any preceding claim, further comprising an annulus running at least partially through the crown and covering a terminal end of the crown.

4. The end effector of any preceding claim, further comprising a first single axis sensor attached to the crown, and/or a second single axis sensor disposed within one spine of the plurality of spines, and/or a dual axis sensor, a flex circuit, or a triple axis sensor disposed proximal the proximal end.

5. The end effector of any preceding claim, a proximal end of each spine comprising a tab that engages in a complementarily shaped recess in a spine coupler.

6. The end effector of any preceding claim, further comprising a wire loop extending along each of the plurality of spines to define a magnetic field location sensor for each spine or at least one wire looping helically about each of the plurality of spines to define a magnetic field location sensor.

7. A housing assembly for a medical device, the housing assembly comprising:
a shell defining a cavity;
an actuator slidable within the cavity along a longitudinal axis and configured to move an end effector between an expanded configuration and a collapsed configuration;
a detent engaged with one of the shell and the actuator; and
a first catch integrated with the other of the shell and the actuator and configured to selectively engage the detent, the engagement of the detent and the first catch providing tactile feedback regarding a collapsed configuration, an intermediate configuration, or an expanded configuration of the end effector.

8. The housing assembly of claim 7, further comprising a second catch integrated with the other of the shell and the actuator and configured to selectively engage the detent.

9. The housing assembly of claim 8, further comprising a third catch integrated with the other of the shell and the actuator and configured to selectively engage the detent.

10. The housing assembly of claim 9, the first catch being configured to provide tactile feedback that the end effector is in the collapsed configuration, the second catch being configured to provide tactile feedback that the end effector is in the intermediate configuration, and the third catch being configured to provide tactile feedback that the end effector is in the expanded configuration.

11. The housing assembly of any of claims 7 to 10, the detent being movable towards and away from the first catch in a direction perpendicular to the longitudinal axis to adjust a force applied by the detent to the first catch.

12. A medical device comprising:
a first shaft extending along a longitudinal axis and attached to an actuator;
an end effector attached to the first shaft at a proximal end of the end effector and configured to move between an expanded configuration, an intermediate configuration, and a collapsed configuration; and
a housing assembly comprising:
an actuator attached to the first shaft and slidable along the longitudinal axis, sliding movement of the actuator sliding the first shaft and the proximal end of the end effector;
a detent; and
a first catch configured to selectively engage with the detent, the first catch being configured to provide tactile feedback that the end effector is in one of the expanded configuration, the intermediate configuration, and the collapsed configuration.

13. The medical device of claim 12, the end effector comprising a plurality of spines, each spine comprising a plurality of electrodes attached thereto.

14. The medical device of claim 12 or claim 13, the first catch comprising a pair of ribs defined on a surface of the actuator.

15. The medical device of any of claims 12 to 14, further comprising a second shaft extending along the longitudinal axis and attached to the housing assembly and a distal end of the end effector, optionally the proximal end of the end effector being configured to slide along the second shaft when moving between the expanded configuration, the intermediate configuration, and the collapsed configuration.
